# FASCICULE DE BREVET EUROPEEN

(11) **EP 3 691 571 B1**
(45) Date de publication et mention de la délivrance du brevet: **28.04.2021**
(21) Numéro de dépôt: 18774079.0
(22) Date de dépôt: 01.10.2018
(51) Int. Cl.: A61F 2/16

(54) **ENSEMBLE CONSTITUÉ D'UNE PAIRE D'IMPLANTS OCULAIRES MULTIFOCAUX**
ANORDNUNG AUS EINEM PAAR VON MULTIFOKALEN OKULARIMPLANTATEN
ASSEMBLY CONSISTING OF A PAIR OF MULTIFOCAL OCULAR IMPLANTS

(30) Priorité: 05.10.2017 FR 1759329
(43) Date de publication de la demande: 12.08.2020
(73) Titulaire: Cristalens Industrie, 22300 Lannion (FR)
(72) Inventeur: CASTIGNOLES, Fannie, 22300 Tredrez-Locquemeau (FR); DELAGE, Denis, 35440 Dinge (FR)
(74) Mandataire: Regimbeau
(86) Numéro de dépôt international: PCT/EP2018/076656
(87) Numéro de publication internationale: WO 2019/068645

(56) Documents cités:
- WO-A2-2009/027438
- US-A- 5 443 507
- US-A1- 2015 320 547

## Description

### DOMAINE DE L'INVENTION

La présente invention se rapporte à un ensemble constitué d'une paire d'implants oculaires multifocaux.

### ARRIERE PLAN TECHNOLOGIQUE DE L'INVENTION

Depuis de nombreuses années, on propose aux personnes souffrant d'une opacification du cristallin (cataracte) de procéder à la destruction et à l'enlèvement de celui-ci, et à son remplacement par un implant intraoculaire.

Ainsi, il est classique de positionner sur les deux yeux d'un patient presbyte (qu'il soit emmétrope ou amétrope) deux implants intraoculaires (implants de sac cristallinien, de chambre antérieure ou intra-cornéens) avec des surfaces optiques multifocales (réfractives ou préférentiellement diffractives).

Le choix de l'implant peut être fait en fonction des habitudes de vie du patient et de ses attentes. On privilégie généralement la vision de loin (par exemple dans une situation de conduite de véhicule) et la vision de près (par exemple dans une situation de lecture d'un livre). Mais avec l'usage toujours croissant des ordinateurs, des tablettes et des smartphones, on cherche aussi à privilégier la vision intermédiaire (illustrée notamment par une situation dans laquelle on consulte un écran).

Un implant avec des surfaces optiques multifocales (réfractives ou préférentiellement diffractives) peut être caractérisé par sa courbe de TFMTF (acronyme anglo-saxon de "Modulation Transfer Function Through Focus" qui peut être traduit par "fonction de transfert de modulation en fonction de la distance de vision ").

Une telle courbe représente la qualité de l'optique (en pourcentage - ou sous forme de ratio compris entre 0 et 1- de contraste de l'image pour un objet de départ ayant un contraste de 100%) en fonction de la distance de vision (laquelle est décrite en addition en Dioptries : 0D = vision de loin ; +1 à +2D = addition intermédiaire ; 3D = addition de près).

De telles courbes sont simulées avec un logiciel de simulation optique tel que le logiciel Zemax (marque déposée) de la société du même nom pour des implants intraoculaires placés dans un modèle d'oeil moyen.

Une courbe de TFMTF est établie pour une fréquence spatiale donnée. Usuellement pour les implants multifocaux, on s'intéresse à la TFMTF à 50 cycles/mm. Mais des TFMTF à 25 cycles/mm (objets plus grossiers) et 100cycles/mm (objets plus fins) sont également intéressants.

Une courbe de TFMTF à 50cycles/mm (par exemple) d'un profil optique donné dépend de la pupille du système optique et de la longueur d'onde de la lumière utilisée.

Ainsi, on peut s'intéresser préférentiellement à une longueur d'onde correspondant à la couleur verte (546nm), mais il peut également être intéressant de tracer la courbe de TFMTF photopique correspondant à l'intégrale des longueurs d'onde de la lumière du jour, ainsi qu'à la courbe de TFMTF scotopique (vision de nuit).

De même, on s'intéresse préférentiellement à une pupille de 3mm de diamètre (correspondant à une vision bien éclairée telle qu'une situation de lecture ou de vision de jour), mais la complémentarité de TFMTF peut être intéressante pour des pupilles de 2 à 6 mm de diamètre.

Une valeur de TFMTF supérieure ou égale à 0.15 est considérée comme apportant une vision proche satisfaisante au porteur, tandis qu'une valeur de TFMTF supérieure ou égale à 0.30 est considérée comme apportant une vision de loin satisfaisante au porteur.

La vision de près "VP" est typiquement +3D (en addition plan cornée), mais peut être comprise entre +2D et +4D.

La vision intermédiaire "VI" est typiquement +1.5D (en addition plan cornée) mais peut être comprise entre +1D et +2D.

Si les courbes de TFMTF d'un implant placé dans un œil permettent de donner une information sur la qualité optique de l'implant, la grandeur physique qui traduit la bonne vision du porteur est son acuité visuelle.

L'acuité visuelle dépend de la TFMTF de l'implant, mais également d'autres paramètres tels que la sensibilité au contraste du porteur. En vision binoculaire, l'acuité visuelle dépend également du traitement neuronal du patient qui combine les informations provenant de ses deux yeux.

Ainsi, même s'il n'est pas possible de simuler directement l'acuité visuelle(AV) d'un porteur en fonction de l'implant utilisé, il est connu que la courbe d'AV a une forme qui suit celle de la courbe de TFMTF, mais avec une enveloppe plus large.

En vision binoculaire, la superposition de deux pics présents sur les courbes de TFMTF pour une même addition permet d'améliorer légèrement l'acuité visuelle.

Ainsi, par exemple, une acuité visuelle de près 8/10 sur un œil et de 8/10 sur l'autre œil peut donner une acuité visuelle binoculaire de 10/10).

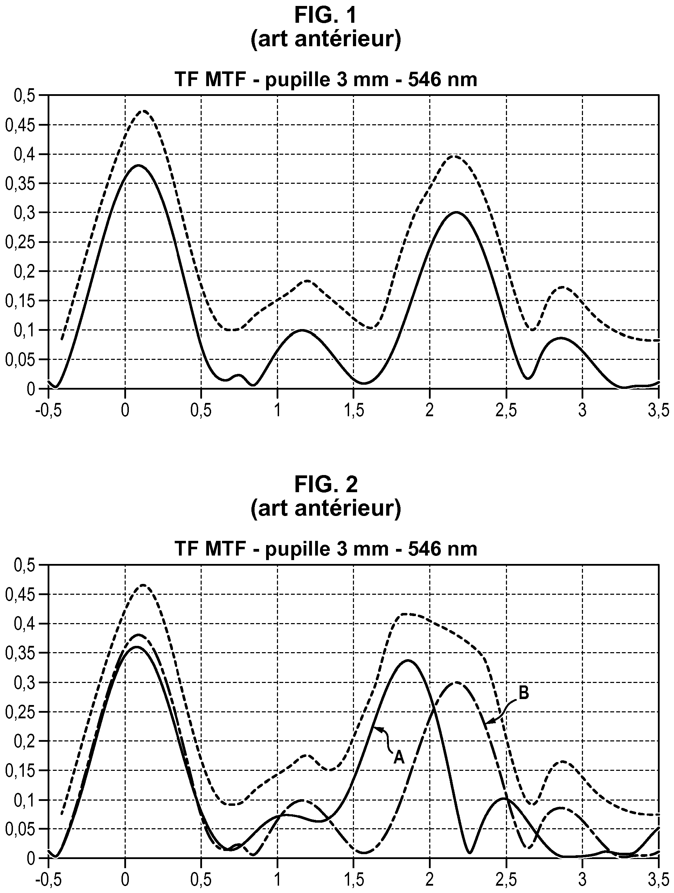

Ceci est illustré à la figure 1 annexée, dans laquelle sont représentées en trait continu les courbes superposées de TFMTF de deux implants identiques d'addition +3D, pour une pupille de 3 mm de diamètre et à une longueur d'onde de 546 nm (en abscisse: addition exprimée en Dioptries /en ordonnée TFMTF à 50 cycles/mm), tandis qu'est représentée en traits discontinus l'estimation de la courbe de TFMTF en vision binoculaire.

En revanche, lorsque les deux yeux présentent un pic de TFMTF légèrement décalé (par exemple avec des implants d'additions de +2.5D et +3D respectivement), l'AV binoculaire cumule les pics en donnant 8/10 à 2.5D et 8/10 à 3D. C'est le principe bien connu du "Mix and Match" en implants intraoculaires où deux implants identiques mais avec une addition différente (même forme de courbe mais pics de vision proche décalés) sont utilisés.

Ceci est illustré à la figure 2 annexée dans laquelle les paramètres sont les mêmes que ceux employés ci-dessus en référence à la figure 1, les courbes caractéristiques des deux implants étant référencées A et B.

Mais la limite de cette pratique est que l'écart entre les deux sommets des pics de VI (vision intermédiaire) et VP (vision de près) doit être limité pour être bien toléré par le porteur. Cet écart est usuellement à 0.5D voire 0.75D (pour des additions autour de 2.5D), car la zone de recouvrement entre les deux courbes en vision proche est faible.

Lorsque les deux yeux ont des implants avec des écarts d'addition supérieurs à 0.75D, il y a, chez la plupart des porteurs, un effet suppresseur (l'œil "défocalisé" ayant donc une TFMTF très basse) en VI qui va faire diminuer le pic de VI de l'œil adapté, et vice versa).

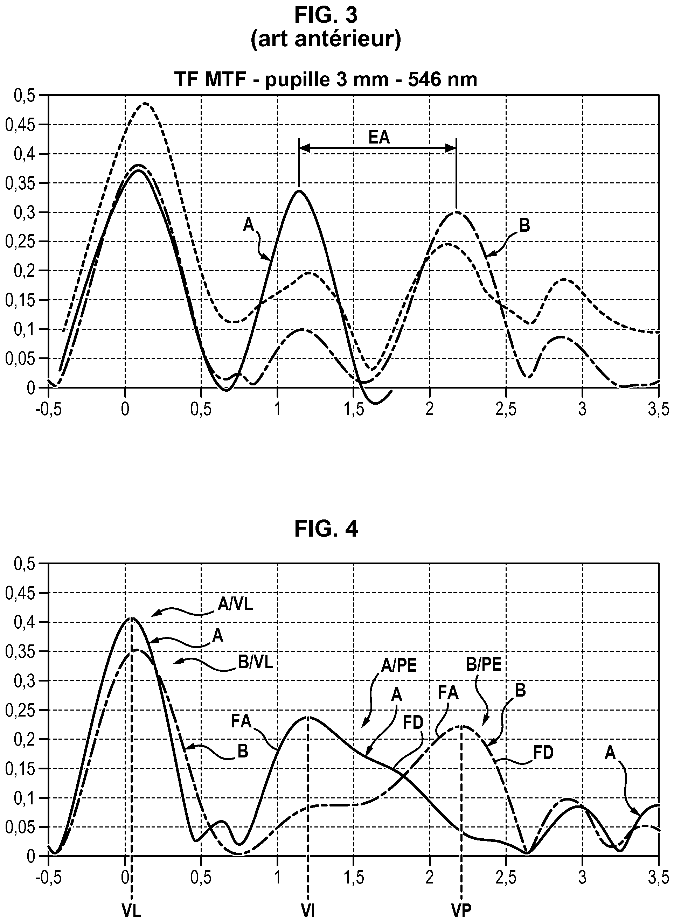

A nouveau, cette situation est illustrée à la figure 3 dans laquelle les paramètres sont les mêmes que ceux employés ci-dessus en référence à la figure 2, l'écart trop grand entre les sommets des pics étant référencé EA.

WO 2009/027438 décrit une paire d'implants oculaires conforme au préambule de la revendication 1 annexée.

Un art antérieur supplémentaire est constitué par les documents US 5 443 507 et US 2015/320547.

La présente invention vise à pallier les difficultés exposées plus haut, en proposant des implants multifocaux qui, lorsqu'ils sont portés ensemble, permettent non seulement au porteur de disposer d'une bonne vision de loin, mais également de bénéficier d'une profondeur de champ continue en vision binoculaire entre la vision intermédiaire et la vision de près.

### RESUME DE L'INVENTION

Ainsi, la présente invention est relative à un ensemble constitué d'une paire d'implants oculaires multifocaux,
- chaque implant de cette paire présentant une courbe de TFMTF ("Modulation Transfer Function Through Focus"/ "fonction de transfert de modulation en fonction de la distance de vision "), pour une pupille de diamètre inférieur ou égal à 4 mm, de préférence inférieur ou égal à3 mm, qui présente un pic correspondant à la vision de loin du porteur, ainsi qu'un pic asymétrique qui s'étale entre la vision intermédiaire et la vision de près, c'est à dire sans discontinuité entre la vision intermédiaire et la vision de près,
caractérisé par le fait que :
- pour un premier implant de cette paire, la valeur de TFMTF est supérieure en vision intermédiaire à celle de la vision de près ;
- pour le second implant de cette paire, la valeur de TFMTF est supérieure en vision de près à celle de la vision intermédiaire ;
le flan ascendant du pic asymétrique du premier implant présente, en valeur absolue, une pente moyenne supérieure à celle de son flan descendant, tandis que le flan ascendant du pic asymétrique du second implant présente, en valeur absolue, une pente moyenne inférieure à celle de son flan descendant.

Par l'expression "pente moyenne", on entend, dans l'ensemble de la présente description et des revendications, la pente de la droite fictive qui passe par le point le plus haut et le point le plus bas du flan correspondant.

Le principe de la présente invention est de combiner sur les deux yeux d'un patient presbyte (qu'il soit emmétrope ou amétrope) deux implants oculaires (implants de sac cristallinien, de chambre antérieure ou intra-cornéens) avec des surfaces optiques multifocales (réfractives ou préférentiellement diffractives) différentes donnant des courbes de fonction de transfert de modulation en fonction de la distance de vision (TFMTF) dites « complémentaires ».

Grâce à la présente invention, on donne au porteur de tels implants une profondeur de champ continue en vision binoculaire entre la vision intermédiaire et la vision de près, qui se traduit par une acuité visuelle supérieure à 5 /10 sur toute cette zone.

Selon d'autres caractéristiques non limitatives et avantageuses de l'invention :
- le croisement desdits pics asymétriques correspond à une valeur de TFMTF d'au moins 0.10 ;
- le croisement desdits pics asymétriques correspond à une valeur de TFMTF d'au moins 0.15 ;
- le sommet de chaque pic asymétrique présente une valeur de TFMTF au moins égale à 0.15 ;
- les valeurs respectives de TFMTF aux sommets des pics correspondant à la vision de loin présentent un écart inférieur à 30%.

### BREVE DESCRIPTION DES DESSINS

D'autres caractéristiques et avantages de l'invention apparaîtront à la lecture de la description suivante d'un mode de réalisation préféré de l'invention. Cette description est faite en référence aux dessins annexés dans lesquels :
- les figures 1 à 3, comme précisé plus haut, illustre l'état de la technique dans le domaine de l'invention ;
- la figure 4 est une représentation, sur un même et unique schéma des courbes de TFMTF de deux implants constituant un ensemble selon l'invention, pour une pupille de 3 mm de diamètre et à une longueur d'onde de 546 nm (en abscisse: addition exprimée en Dioptries /en ordonnée TFMTF à 50 cycles/mm) ;
- la figure 5 est une vue identique à la précédente sur laquelle est portée en plus, l'estimation de la courbe de TFMTF en vision binoculaire ;
- la figure 6 est une courbe de TFMTF en vision binoculaire d'implants appartenant à l'art antérieur.

### DESCRIPTION DETAILLEE DE L'INVENTION

Ainsi que cela a été précisé plus haut, la présente invention découle du fait que l'intérêt de disposer de la combinaison de deux TFMTF dites « complémentaires » est de permettre de mieux fusionner la vision des deux yeux du porteur des implants, tout en autorisant un écartement plus important entre les deux sommets des pics de VI (vision intermédiaire) et VP (vision proche) qu'avec les dispositifs antérieurs cités plus haut qui font application du principe "Mix and Match".

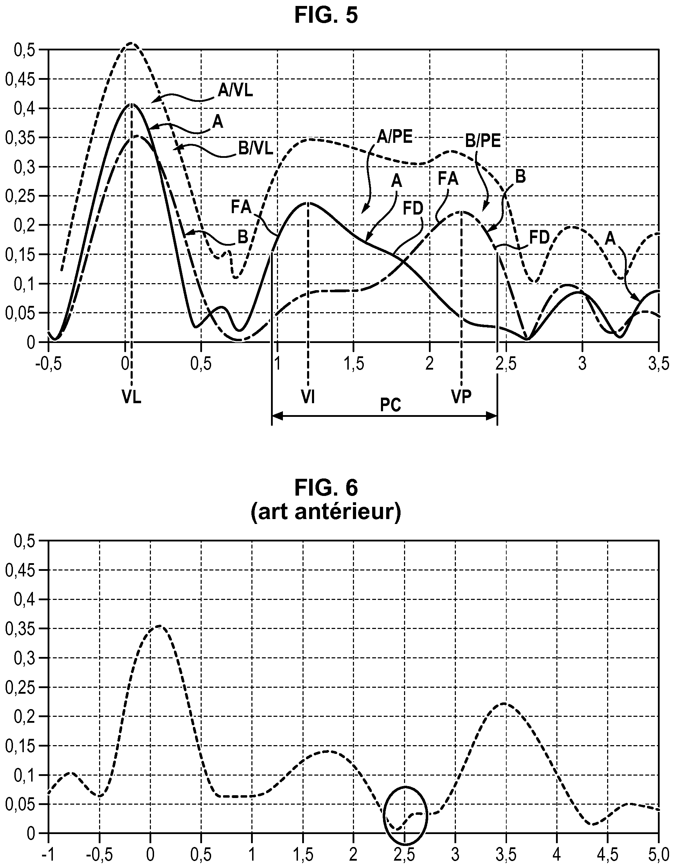

On se reportera aux figures 4 et 5 annexées pour décrire les courbes préférées de TFMTF des deux implants constitutifs de l'ensemble selon l'invention.

Sur ces figures, les courbes respectives de TFMTF de chacun des deux implants sont référencées A et B et correspondent par exemple à l'œil dominant, respectivement l'œil dominé du porteur (ou inversement).

On note en premier lieu que chaque courbe A et B présente un pic A/VL, respectivement B/VL correspondant à la vision de loin du porteur (avec correction des erreurs réfractives si besoin.

Ces pics sont similaires pour les deux implants. En tout état de cause, les valeurs respectives de TFMTF aux sommets des pics A/VL et B/VL présentent un écart inférieur à 20% et de préférence inférieur à 10%.

Par ailleurs, les deux courbes A et B présentent un "pic élargi asymétrique" A/PE, respectivement B/PE (par rapport à un multifocal classique) qui s'étale entre la vision intermédiaire VI et la vision de près VP. En d'autres termes, on ne distingue pas deux pics distincts, mais au contraire, il y a continuité de la TFMTF entre la VI et la VP) tout en conservant un niveau minimal de 0.10, préférentiellement 0.15.

De plus, pour un premier implant de l'ensemble qui correspond à la courbe A, la valeur de TFMTF est supérieure en vision intermédiaire VI à celle de la vision de près VP.

Et a contrario, pour le second implant de l'ensemble qui correspond à la courbe B, la valeur de TFMTF est supérieure en vision de près VP à celle de la vision intermédiaire VI.

Enfin, les deux courbes se complémentent de sorte qu'elles présentent un profil asymétrique avec une « pente globale » plus douce dans leur zone de croisement.

Autrement exprimé, le flan ascendant FA du pic asymétrique A/PE du premier implant présente, en valeur absolue, une pente moyenne supérieure à celle de son flan descendant FD, tandis que le flan ascendant FA du pic asymétrique B/PE du second implant présente, en valeur absolue, une pente moyenne inférieure à celle de son flan descendant FD.

Avantageusement, les deux courbes A et B de TFMTF se croisent à un seuil supérieur ou égal à 0.15.

Ainsi, en vision binoculaire, le porteur de la paire d'implants de l'ensemble selon l'invention possède une TFMTF supérieure à 0.15 sur une plage de distance ou profondeur de champ (EDOF) beaucoup plus étendue, typiquement de 1D à 2.5D (ce qui correspond à une distance de vision de 40 cm à 1 m).

Comparativement, des systèmes "Mix and Match" (M&M) de l'état de la technique offrent respectivement une vision nette de 40 cm à 66 cm -voire 75 cm- respectivement en M&M +2.5D/+3D et M&M +2.25D/+3D.

De plus, la combinaison de deux TFMTF dites « complémentaires » apporte également une vision binoculaire plus intéressante que celle d'un implant trifocal possédant un pic en vision de loin VL, un pic en vision intermédiaire VI, et un pic en vision de près VP, avec des annulations de la TFMTF entre chacun de ces pics, ce qui se traduit par une absence de vision continue entre la vision de près VP et la vision intermédiaire VI. Cela est illustré à la figure 6 ou la zone d'annulation de la TFMTF est repérée par un cercle.

La profondeur de champ PC de la TFMTF binoculaire permet une continuité entre sa vision de près et intermédiaire sans chute de netteté entre les deux, ainsi que cela apparaît sur la courbe en traits interrompus de la figure 5.

De manière bien connue, l'un des principaux inconvénients associés aux implants multifocaux est la présence de halos.

Ainsi, avec un implant bifocal, en vision nocturne, et principalement lors de la conduite (c'est à dire lorsque la vision "utile" est la vision de loin), le porteur peut être gêné par le halo créé par le pic de la vision de près VP.

En revanche, avec un implant trifocal, la vision de loin est associée à deux halos correspondant aux pics de la vision de près et de la vision intermédiaire. Cependant, la hauteur de ces deux pics étant plus faible, l'effet de ces halos est moins gênant pour le porteur qu'avec un implant bifocal classique.

En d'autres termes, le porteur supporte mieux plusieurs halos plus faibles plutôt qu'un halo fort et bien délimité.

Ainsi, l'utilisation de deux implants « complémentaires » selon l'invention permet de créer un halo continu ou diffus qui est moins gênant en condition de vision de loin et tout particulièrement de conduite de nuit.

L'homme du métier procèdera à la fabrication des deux implants intraoculaires (implants de sac cristallinien, de chambre antérieure ou intra-cornéens) avec des surfaces optiques multifocales (réfractives ou préférentiellement diffractives) différentes, afin que ceux-ci présentent les caractéristiques exprimées plus haut.

Comme pour des implants de l'art antérieur, ces implants peuvent être mis en place sur les yeux d'un patient grâce à un injecteur. Et lorsque cette mise en place est réalisée dans le sac cristallinien, ils sont introduits en position repliée à travers une fente de petite dimension prévue à cet effet.

Bien que la présente description ait été faite en relation avec des implants intraoculaires, la présente invention s'applique également aux implants intra cornéens et aux "piggyback", c'est à dire aux implants mis en place en avant du sac cristallinien.

## Revendications

1. Ensemble constitué d'une paire d'implants oculaires multifocaux, :
- chaque implant de cette paire présentant une courbe (A,B) de TFMTF ("Modulation Transfer Function Through Focus"/ "fonction de transfert de modulation en fonction de la distance de vision "), pour une pupille de diamètre inférieur ou égal à 4 mm, de préférence inférieur ou égal à 3 mm, qui présente un pic (A/VL ; B/VL) correspondant à la vision de loin (VL) du porteur, ainsi qu'un pic asymétrique (A/PE ; B/PE) qui s'étale entre la vision intermédiaire (VI) et la vision de près (VP), c'est à dire sans discontinuité entre la vision intermédiaire (VI) et la vision de près (VP),
**caractérisé par le fait que** :
- pour un premier implant de cette paire, la valeur de TFMTF est supérieure en vision intermédiaire (VI) à celle de la vision de près (VP);
- pour le second implant de cette paire, la valeur de TFMTF est supérieure en vision de près (VP) à celle de la vision intermédiaire (VI) ;
- le flan ascendant (FA) du pic asymétrique (A/PE) du premier implant présente, en valeur absolue, une pente moyenne supérieure à celle de son flan descendant (FD), tandis que le flan ascendant (FA) du pic asymétrique (B/PE) du second implant présente, en valeur absolue, une pente moyenne inférieure à celle de son flan descendant (FD).

2. Ensemble selon la revendication 1, **caractérisé par le fait que** le croisement desdits pics asymétriques (A/PE ; B/PE) correspond à une valeur de TFMTF d'au moins 0.10.

3. Ensemble selon la revendication 2, **caractérisé par le fait que** le croisement desdits pics asymétriques (A/PE ; B/PE) correspond à une valeur de TFMTF d'au moins 0.15.

4. Ensemble selon la revendication 3, **caractérisé par le fait que** le sommet de chaque pic asymétrique (A/PE ; B/PE) présente une valeur de TFMTF au moins égale à 0.15.

5. Ensemble selon l'une des revendications précédentes, **caractérisé par le fait que** les valeurs respectives de TFMTF aux sommets des pics (A/VL ; B/VL) correspondant à la vision de loin (VL) présentent un écart inférieur à 30%.

## Patentansprüche

1. Anordnung, die aus einem Paar von multifokalen Okularimplantaten besteht:
- wobei jedes Implantat dieses Paares eine TFMTF -Kurve (A, B) (Through Focus Modulation Transfer Function / Modulationstransferfunktion in Abhängigkeit von der Sehweite) für eine Pupille mit einem Durchmesser von kleiner oder gleich 4 mm, vorzugsweise kleiner oder gleich 3 mm, aufweist, die einen Scheitel (A/VL; B/VL), der der Fernsicht (VL) des Trägers entspricht, sowie einen asymmetrischen Scheitel (A/PE; B/PE) aufweist, der sich zwischen der Zwischensicht (VI) und der Nahsicht (VP) erstreckt, das heißt, ohne Diskontinuität zwischen der Zwischensicht (VI) und der Nahsicht (VP),
**dadurch gekennzeichnet, dass**:
- für ein erstes Implantat dieses Paares, der TFMTF -Wert bei der Zwischensicht (VI) höher als derjenige der Nahsicht (VP) ist;
- für das zweite Implantat dieses Paares, der TFMTF -Wert bei der Nahsicht (VP) höher ist als derjenige der Zwischensicht (VI);
- die ansteigende Flanke (FA) des asymmetrischen Scheitels (A/PE) des ersten Implantats als Absolutwert eine mittlere Steigung aufweist, die größer als diejenige seiner absteigenden Flanke (FD) ist, während die ansteigende Flanke (FA) des asymmetrischen Scheitels (B/PE) des zweiten Implantats als Absolutwert eine mittlere Steigung aufweist, die kleiner als diejenige seiner absteigenden Flanke (FD) ist.

2. Anordnung nach Anspruch 1, **dadurch gekennzeichnet, dass** die Überschneidung der asymmetrischen Scheitel (A/PE; B/PE) einem TFMTF -Wert von mindestens 0,10 entspricht.

3. Anordnung nach Anspruch 2, **dadurch gekennzeichnet, dass** die Überschneidung der asymmetrischen Scheitel (A/PE; B/PE) einem TFMTF -Wert von mindestens 0,15 entspricht.

4. Anordnung nach Anspruch 3, **dadurch gekennzeichnet, dass** die Spitze von jedem asymmetrischen Scheitel (A/PE; B/PE) einen TFMTF -Wert von mindestens gleich 0,15 aufweist.

5. Anordnung nach einem der vorhergehenden Ansprüche, **dadurch gekennzeichnet, dass** die entsprechenden TFMTF -Werte an den Spitzen der Scheitel (A/VL; B/VL), die der Weitsicht (VL) entsprechen, eine Abweichung von kleiner als 30% aufweisen.

## Claims

1. An assembly consisting of a pair of multifocal ocular implants,
- each implant of this pair having a TFMTF (Modulation Transfer Function Through Focus) curve, for a pupil of diameter less than or equal to 4 mm, preferably less than or equal to 3 mm, which has a peak (A/VL; B/VL) corresponding to the distance vision (VL) of the wearer, as well as an asymmetrical peak (A/PE; B/PE) which spreads between intermediate vision (VI) and near vision (VP), that is to say without discontinuity between intermediate vision (VI) and near vision (VP),
**characterized by** the fact that:
- for a first implant of this pair, the TFMTF value is greater in intermediate vision (VI) than that of near vision (VP);
- for the second implant of this pair, the TFMTF value is greater in near vision (VP) than that of intermediate vision (VI);
- the rising edge (FA) of the asymmetrical peak (A/PE) of the first implant has, as an absolute value, a mean slope greater than that of its falling edge (FD), whereas the rising edge (FA) of the asymmetrical peak (B/PE) of the second implant has, as an absolute value, a mean slope less than that of its falling edge (FD).

2. The assembly according to claim 1, **characterized by** the fact that the overlap of said asymmetrical peaks (A/PE; B/PE) corresponds to a TFMTF value of at least 0.10.

3. The assembly according to claim 2, **characterized by** the fact that the overlap of said asymmetrical peaks (A/PE; B/PE) corresponds to a TFMTF value of at least 0.15.

4. The assembly according to claim 3, **characterized by** the fact that the apex of each asymmetrical peak (A/PE; B/PE) has a TFMTF value at least equal to 0.15.

5. The assembly according to one of the preceding claims, **characterized by** the fact that the respective TFMTF values at the apices of the peaks (A/VL; B/VL) corresponding to distance vision (VL) have a difference of less than 30%.
